# EUROPEAN PATENT SPECIFICATION

(11) **EP 2 152 901 B1**
(45) Date of publication and mention of the grant of the patent: **26.03.2014**
(21) Application number: 08779376.6
(22) Date of filing: 11.06.2008
(51) Int. Cl.: C12Q 1/68

(54) **METHOD FOR INTRODUCING COMMON AND/OR INDIVIDUAL SEQUENCE ELEMENTS IN A TARGET NUCLEIC ACID MOLECULE**
VERFAHREN ZUR EINFÜHRUNG VON GEMEINSAMEN UND/ODER INDIVIDUELLEN SEQUENZELEMENTEN IN EINEM TARGET-NUKLEINSÄUREMOLEKÜL
PROCÉDÉ PERMETTANT D'INTRODUIRE DES ÉLÉMENTS DE SÉQUENCE COMMUNS ET/OU INDIVIDUELS DANS UNE MOLÉCULE CIBLE D'ACIDE NUCLÉIQUE

(30) Priority: 11.06.2007 SE 0701401
(43) Date of publication of application: 17.02.2010
(73) Proprietor: Agilent Technologies, Inc., Santa Clara, CA 95051 (US)
(72) Inventor: JOHANSSON, Henrik, 75232 Uppsala (SE); ERICSSON, Olof, 75233 Uppsala (SE)
(74) Representative: Grünecker, Kinkeldey, Stockmair & Schwanhäusser
(86) International application number: PCT/SE2008/050694
(87) International publication number: WO 2008/153492

(56) References cited:
- WO-A1-91/03573
- WO-A1-2004/094664
- WO-A1-2005/111236
- US-A1- 2003 082 543
- US-A1- 2006 292 597
- DAHL F. ET AL.: 'Multiplex amplification enabled by selective circularization of large sets of genomic DNA fragments' NUCLEIC ACIDS RESEARCH vol. 33, no. 8, 2005, pages 1 - 7, XP003023505
- STAHL R. ET AL.: 'T-DNA integration into the barley genome from single and double cassette vectors' PNAS vol. 99, no. 4, 19 February 2002, pages 2146 - 2151, XP003023506
- LYAMICHEV V. ET AL.: 'Structure-Specific Endonucleotic Cleavage of Nucleic Acids by Eubacterial DNA Polymerases' SCIENCE vol. 260, 07 May 1993, pages 778 - 783, XP002910110

## Description

### Field of invention

The invention is directed to the field of multiplex DNA analysis. It includes a strategy to reduce sample complexity by specifically equipping a definable set of genomic sequences, in a biological sample, with a number of common or individual sequence motifs. This modification of sample DNA includes one sequence specific adaptor ligation followed by one run-off polymerization.

### Background of the invention

The present invention is a contribution to the growing research field of large-scale genetic analysis. In recent years several new strategies for genome sequencing have been presented by companies such as 454 sequencing, Solexa and Helicos. In contrast to Sanger sequencing, which has been and still is the most employed technique, the new approaches aim at recording several thousand spatially resolved sequence reads in parallel from one reaction. Massively parallel DNA sequencing methods have the potential of lowering both the cost and the time per sequenced base by orders of magnitude.

Approaches for selection of sequence subsets from a complex pool of nucleic acids include the following prior art.

PCT WO2005/111236 describes the selector technology, a method where both ends of a genomic single stranded DNA fragment are ligated to either the same oligonucleotide or two separate olionucleotides, called vectors, creating either a closed circular molecule or a linear molecule with common motifs flanking the targeted DNA sequence. The genomic fragments are digested with restriction endonucleases prior to selection to attain a specific 3'-end that can be joined by ligation with the vector. The ligations are templated by a second molecule, called selector, complementary to both ends of the genomic single stranded DNA fragment.

In contrast to the present invention, this method requires that all target molecule 3'-ends are defined by one or more restriction endonucleases. Incorporation of tag sequences into the selected fragments is impractical due to requirement of a plurality of both vector and selector probes. Further on, the targeted region forms a closed circular molecule reducing replication efficiency by topological hindrance.

Willis et al. (US 6,858,412 B2) describes a strategy utilizing open circular probes. These are polynucleotides with ends complementary to two specific parts of the genome spaced by at least one nucleotide gap. The gap is subsequently filled by nucleotide extension and a circular molecule is formed by ligation. For the gapfill reaction to be successful the polymerase need to stop at the exact right position, this is difficult to achieve when large gaps (>50 bases) are to be filled and ligated.

Fredriksson (Nucleic Acids Res, 2007;35(7):e47) describes a method where multiple genomic fragments are amplified with multiple primer pairs for eight cycles under relaxed conditions creating correct and incorrect amplicons. Each correct amplicon is subsequently circularized via a third oligonucleotide targeting the combinations of primer pairs and then amplified with Templiphi (GE). Three unique oligonucleotides are required per target sequence. The method succeeds in amplifying 90% of the targeted molecules but also creates 42% nonspecific products. Employed as a method for complexity reduction this will increase the amount of oversampling needed.

### Summary of the invention

There is a need in the art for methods to further increase the useful information gained from each reaction. The invention thus relates to a strategy useful for preparative complexity reduction of DNA samples. Since only about one percent of the genome consists of open reading frames serving as templates for protein translation, we predict that the majority of applications will benefit from a method for selectively applying tags or universal handles to a subset of the sequences in a sample.

Compared to prior art the major advantages of the current invention is that it enables full freedom in the selection of target sequences along with the possibility to equip the selected sequences with individual artificial tag sequences.

It is our strong conviction that the strategy we herein propose for sequence specific linkage of two motifs to the same molecule that can be carried out in parallel will offer unique advantages over previously described methods. The main advantages of the method are freedom in probe design and possibility to incorporate individual sequence elements, such as tag motifs.

The invention includes means to equip a target nucleic acid molecule population with both common sequence elements and individual sequence elements unique for single targets and or subpopulations within the selected target sequences. The common elements can subsequently be utilized for e.g. amplification of all selected target sequences while the individual sequence elements can be used for e.g. analysis, quantification or sorting of the respective target molecules.

Thus, in a first aspect, the disclosure relates to a method for introducing common and/or individual sequence elements in a target nucleic acid molecule in a sample comprising sample nucleic acid molecules, said method comprising the steps
i) denaturing the sample nucleic acid molecules, if the sample nucleic acid molecules are double-stranded, to obtain single stranded sample nucleic acid molecules;
ii) bringing the sample nucleic acid molecules in contact with primary, secondary and tertiary probe nucleic acid molecules, wherein the 3'-end of the tertiary probe comprises a part complementary to the primary probe and the 5'-end of the tertiary probe comprises a part complementary to a 5'-part of the target nucleic acid molecule; the 3'-end of the secondary probe is complementary to a 3'-part of the target nucleic acid molecule and the 5'-end of the secondary probe is not complementary to the target nucleic acid molecule; wherein said primary, secondary and tertiary probes comprise said common and/or individual sequence elements;
iii) ligating the 3'-end of the primary motif to the 5'-end of the target nucleic acid molecule; and
iv) elongating the 3'-end of the secondary motif by means of a nucleic acid polymerase.

In an alternative aspect, step iv) of the method is modified to step
iv') elongating the 3'-end of the target nucleic acid molecule by means of a nucleic acid polymerase.

In one embodiment of the invention, the 5'-end of the secondary probe is linked to the 3'-end of the tertiary probe, e.g. by a phosphodiester bond so that the two probes constitute a single nucleic acid molecule.

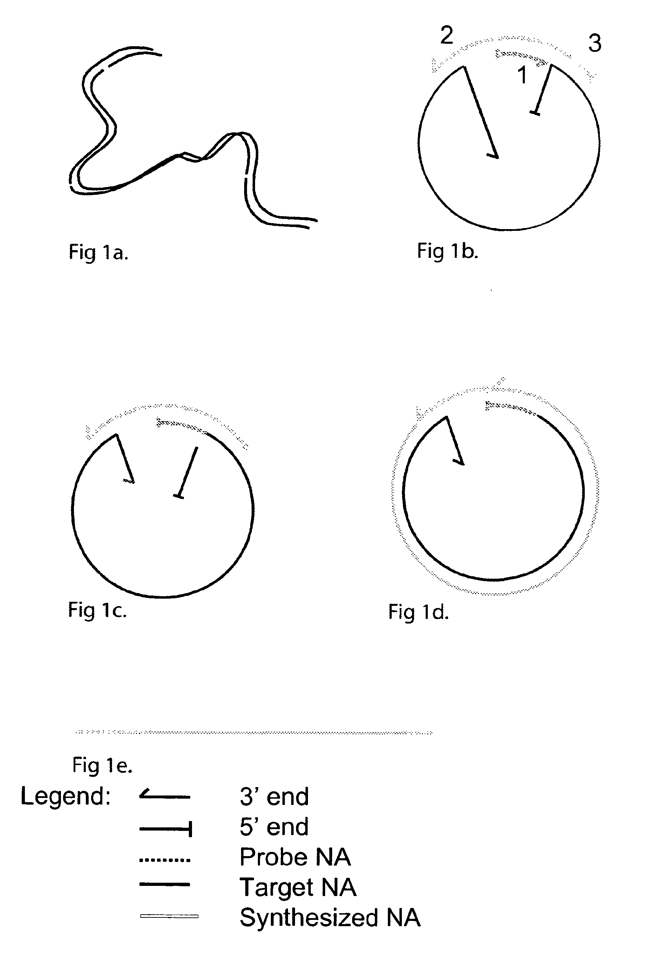

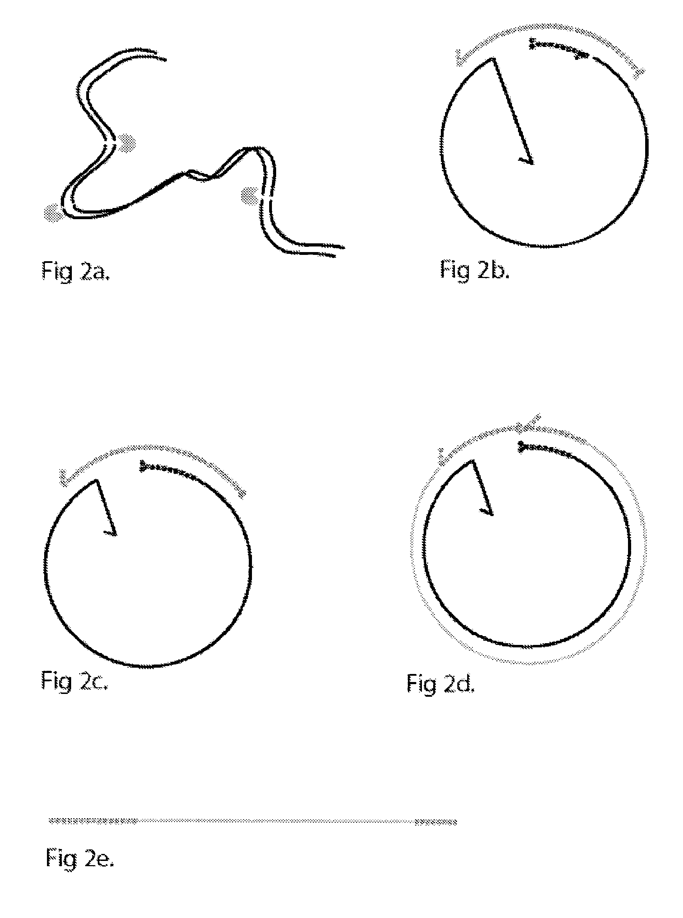

If the 5'-end of tertiary probe is complementary to the sequence of the 5'-end of the target nucleic acid molecule, then the primary probe will be adjacent to the 5'-end of the target nucleic acid molecule and the ligation step iii) may be performed without further steps, cf. Figure 2. However, if the 5'-end of tertiary probe is complementary to a sequence that is in the 5'-part of the target molecule, but not at the 5'-end of the target nucleic acid molecule, then there will be a "flapping 5'-end" of the target nucleic acid molecule that will need to be cleaved off before the ligation step iii), cf. Figure 1. In this case, the method includes a step
iia) cleaving off a part of the 5'-end of the target nucleic acid molecule that is not complementary to the tertiary probe.

The sample nucleic acid molecules may be fragmented before applying the method according to the invention. This may be done by either random fragmentation, such as sonication or treatment with UV-radiation, or in a sequence specific manner, such as by the use of restriction enzymes.

In one embodiment of the method according to the invention, the common and/or individual sequence elements introduced into the target nucleic acid molecules comprise one or several primer binding sites, tag sequences, barcode sequences, spacers, sites for enzymatic restriction digestion, sequence for sizecoding of fragments etc.

In a further aspect, the invention relates to a method for selectively amplifying a target nucleic acid molecule in a sample comprising sample nucleic acid molecules, comprising the steps
- introducing sequence elements comprising amplification primer binding sites in the target nucleic acid molecule with the method according to the invention; and
- amplifying the target nucleic acid molecule.

### Brief description of the drawings

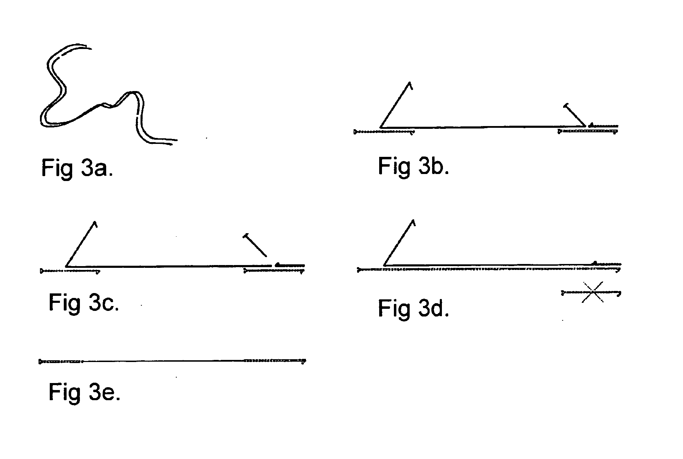

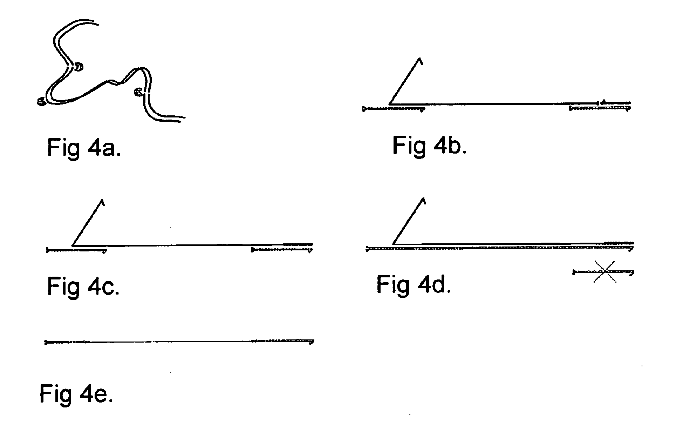

Figure 1:
   a) The genomic sample is subjected to either random fragmentation e.g sonication or UV-treatment.
   b) The primary (1) and the secondary (2) probe are added to the sample to form complex with the target sequence. In this example the secondary probe and the tertiary (3) probe are linked together to form a single polynucleotide.
   c) The flapping 5'-end of the target sequence is cleaved off by structure specific cleavage to create the prerequisite for ligation between the primary probe and the target sequence. A portion of the tertiary probe serves as ligation template.
   d) The 3'-end of the secondary probe is elongated by a DNA polymerase and will thereafter contain the complement sequence of the original target sequence as well as the complement of the primary probe (ligation substrate) (fig 1 e).
   Figure 1 further comprises a legend that is valid for figures 1-6.
Figure 2
   a) The genomic sample is subjected to a sequence specific fragmentation such as a restriction endonuclease cleavage to create a defined 5' end.
   b) The tertiary probe and the primary probe is added to the sample to form complex with the target sequence. In this example the secondary probe and the tertiary probe forms a single polynucleotide.
   c) The target sequence and the primary probe are joined by ligation
   d) The 3'-end of the secondary probe is elongated by a DNA polymerase and will thereafter contain the complement sequence of the original target sequence as well as the complement of the primary probe (fig 2 e).
Figure 3
   a) The genomic sample is subjected to either random fragmentation e.g sonication or UV-treatment.
   b) The primary and the secondary probe along with the tertiary probe are added to the sample to form complex with the target sequence.
   c) The flapping 5'-end of the target sequence is cleaved off by structure specific cleavage to create the prerequisite for ligation between the primary probe and the target sequence. A portion of the tertiary probe serves as ligation template.
   d) The 3'-end of the secondary probe is elongated by a DNA polymerase and will thereafter contain the complement sequence of the original target sequence as well as the complement of the primary probe (ligation substrate) (fig 3e).
Figure 4
   a) The genomic sample is subjected to a sequence specific fragmentation such as a restriction endonuclease cleavage to create a defined 5' end.
   b) The primary and the secondary probe along with the tertiary probe are added to the sample to form complex with the target sequence.
   c) The target sequence and the primary probe are joined by ligation
   d) The 3'-end of the secondary probe is elongated by a DNA polymerase and will thereafter contain the complement sequence of the original target sequence as well as the complement of the primary probe (fig 4 e).
Figure 5
   a) The genomic sample is subjected to a sequence specific fragmentation such as a restriction endonuclease cleavage to create a defined 3'-end.
   b) The primary and the secondary probe along with the tertiary probe are added to the sample to form complex with the target sequence.
   c) The target sequence and the primary probe are joined by ligation. The 3'-end of the target sequence is elongated by a DNA polymerase and the complement sequence of one part of the secondary probe is replicated onto the target sequence.
   d) A molecule containing the targeted sequence flanked by the primary and secondary probe is created.
Figure 6
   a) The genomic sample is subjected to a sequence specific fragmentation such as a restriction endonuclease cleavage to create both a defined 5' and 3' end.
   b) The primary and the secondary probe along with the tertiary probe are added to the sample to form complex with the target sequence.
   c) The target sequence and the primary probe are joined by ligation. The 3'-end of the target sequence is elongated by a DNA polymerase and the complement sequence of one part of the secondary probe is replicated onto the target sequence.
   d) A molecule containing the targeted sequence flanked by the primary and secondary probe is created.

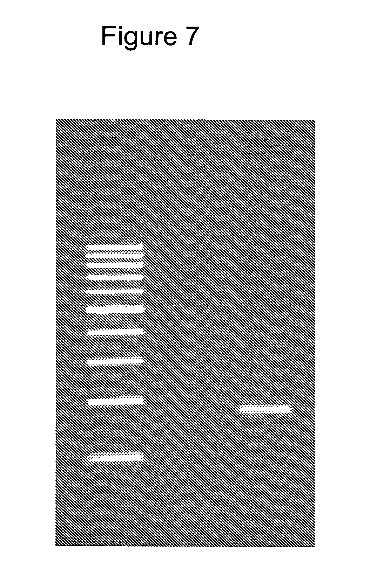

Figure 7: Agarose gel from Example, step 5.

### Definitions

All words and terms used in the present specification are intended to have the meaning usually given to them by the person skilled in the art. For sake of clarity some terms are explicitly defined below.

If not otherwise indicated, when nucleic acid molecules are mentioned in singular form in this specification, this is intended to mean all the nucleic acid molecules of a singular sequence, not a singular molecule. Correspondingly, when nucleic acid molecules are referred to in the plural this is intended to mean nucleic acid molecules of different sequences.

*Target nucleic acid molecule:* A nucleic acid molecule, comprised in a sample, that it is of interest to introduce sequence elements in. A DNA or RNA molecule comprising at least two segments with known sequence having segments with known or unknown sequence between them.
*Target nucleic acid set:* A plurality of target molecules.
*Sample nucleic acid molecules:* The nucleic acid molecules comprised in a sample. Sample nucleic acid molecules may be both DNA or RNA.
*Sample:* Any sample comprising nucleic acid molecules of interest.
*Probe nucleic acid molecule:* Nucleic acid molecules of known sequence used to introduce the common and individual sequence elements. The probe nucleic acid molecules of the present invention comprise three distinct regions called primary, secondary and tertiary motifs. The primary motif is contained in a separate probe molecule, called the primary probe, while the secondary and tertiary motifs may be on the same or separate probe molecules, called secondary and tertiary probes.

*Common sequence element:* A nucleic acid molecule element with a sequence common to all primary, secondary and/or tertiary probe nucleic acid molecules.
*Individual sequence element* A nucleic acid molecule element with a sequence unique to a probe nucleic acid molecule or a subset of all probe nucleic acid molecules.
*Tag sequence:* A nucleic acid molecule segment that can be used for directed hybridization to a complementary polynucleotide in solution or on solid phase.
*Primary probe:* A polynucleotide which in the method is linked to the 5'-end of the target sequence by ligation. The primary probe is partially complementary to the tertiary probe.
*Secondary probe:* A polynucleotide the 3'-end of which is complementary to the target nucleic acid molecule and therefore capable to prime elongation on the target sequence. It's 5'-end may be linked, by for example a phosphodiester bond, to the 3'-end of the tertiary motif.
*Tertiary probe:* A polynucleotide comprising at least one part complementary to a target nucleic acid molecule and one part complementary to the primary motif.

### Detailed description of the invention

The main goal of the proposed application is to equip both ends of a subset of sample nucleic acid molecules in a complex sample, that is target nucleic acid molecules, with common or individual sequence elements. These sequence elements can then be used for a variety of applications. The common element or elements can be used to address the complete subset, e.g. by PCR, and individual elements can be used for sorting amplicons or balancing individual sequence frequencies within the population.

The invention includes means to equip a set of target nucleic acid molecules within a plurality of other nucleic acid molecules, sample nucleic acid molecules, with common motifs on both sides of the target molecules at specific sites. The method enables discrimination of a set of target molecules from other nucleic acid molecules present in a biological sample. In the preferred embodiment the flanking sequences are subsequently utilized to specifically amplify the selected target set by PCR but other embodiments include separate nucleic acid amplification mechanisms and or analysis approaches.
The invention associates at least one defined sequence motif with each of the two ends of the target molecule. This is achieved through the combination of two separate mechanisms. The 5'end of the target molecule is associated with a defined sequence by a templated ligation involving the primary and the tertiary probe. At the target molecules 3'-end, replication primed by the secondary probe is initiated. This replication results in a molecule containing from 5' to 3' : The secondary probe, the complement of the target sequence and the complement sequence of the primary probe.

### Sample preparation

One of the main advantages with the current invention is the freedom of design. This freedom is achieved at the 5'-end of the target molecule by using a Structure-specific endonucleolytic cleavage to cleave (Lyamichev et al Science. 1993 May 7;260(5109):778-83) off the flapping 5'-end (fig 1c, 3c, and 5c) and at the 3'-end by simply alleviating the target molecules 3'-end in the priming reaction (part d of fig 1-6). However depending on the source of nucleic acids, the sample may have to be prepared to some extent. If genomic DNA is used as source DNA it will advantageously be fragmented to achieve complete denaturation of the double stranded DNA into single stranded DNA available to probe hybridization. Besides this, fragmentation of the DNA will also increase diffusion rate and speed up reaction kinetics, reduce secondary structures and increase invasive cleavage reaction efficiency. The fragmentation does not have to generate a specific pair of ends of the target molecule but may be performed by approaches that fragment DNA randomly such as sonication or incomplete DNAse treatment. However specific embodiments (fig 2,4,5,6) may use sequence specific cleavage of the sample nucleic acid, such as restriction enzyme cleavage to define at least one end of the target molecule.

If sequence independent nucleic acid fragmentation is utilized, the 5' end of the target nucleic acid will be defined by structure specific endonucleolytic cleavage subsequent to hybridization to the tertiary probe and in complex with the primary probe (fig 1b-c). The 3' end will be defined by the polymerization initiating from the secondary probe, subsequently also incorporating the ligation complement-into the same molecule as the secondary probe and the target.

### Association of a 5' primary motif

The different variants of this step are depicted in fig 1-6 b and c. The first step of the method involves the linking of the primary probe to the 5' end of the target sequence. This is achieved by ligation of the primary probe to the 5' end of the target sequence. The reaction is templated by the tertiary probe which is complementary to both the target molecule and the primary probe. If a sequence independent fragmentation is used such as sonication, the 5' end of the target sequence needs to be cleaved to form a junction suitable for ligation before association of the primary probe and the target sequence can occur. This can be achieved by structure specific cleavage carried out by for example taq polymerase or fen-1 (see figure 1b). The use of this reaction step enables introduction of a cleavage site at any position of the target molecule's 5'-end. The structure specific cleavage leaves a nick-structure that can be ligated by for example a DNA ligase. After this step has occured, the introduction of a predefined motif to the target sequence 5' end is completete.

### Association of a 3' secondary motif

The different variants of this step are depicted in fig 1-6 d. To assign a predefined motif to the 3' end of the target molecule, a polynucleotide herein referred to as the secondary probe is used. The secondary probe comprises sequences to be used in downstream applications along with at least one target complementary sequence separated by a distance on the target molecule ranging from 1-10000 bp from where the primary probe is positioned.

The part of the secondary probe not complementary to the target can comprise functional motifs such as primer binding sites and/or tag elements for array sorting. The secondary probe 3' end is complementary to the 3'-part of the target molecule. Upon polymerization initiated from the secondary probe 3' end, templated by the target molecule, a polynucleotide is created containing the secondary motif followed by the complement to the target sequence and finally the complement to the primary motif including the secondary motif.

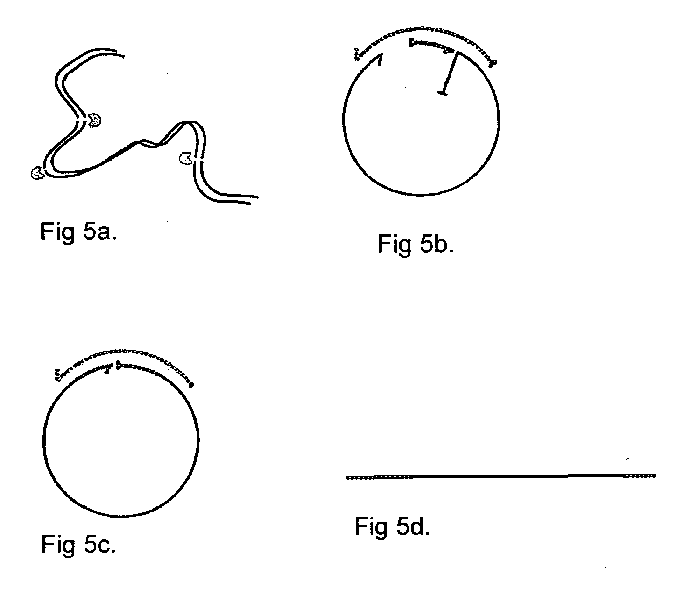

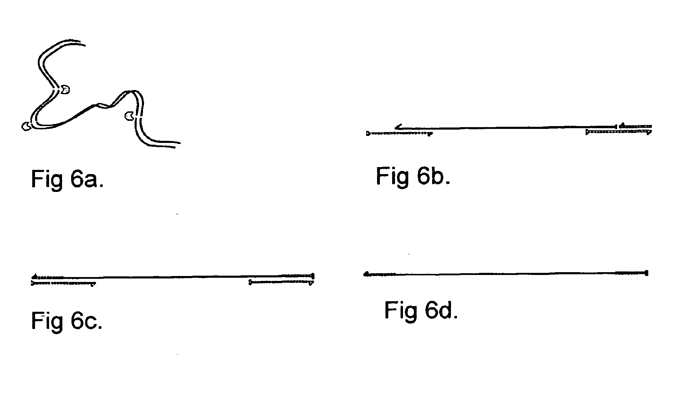

One embodiment of the invention includes utilizing the secondary probe as template for polymerization primed from the 3' end of the target sequence (figures 5 and 6). However, depending on assay design and polymerase, this may require a predefined target sequence 3' end.

In the primary embodiment of the invention the secondary probe and the tertiary probe are linked by a covalent bond, preferentially a phosphodiesterbond, intramolecularly linking the ligation and polymerization. However, also other association types can be envisioned.

The reaction can be carried out on a plurality of target sequences in the same reaction and when the two motifs have been associated with the target sequences the molecules may be subjected to for example reaction steps requiring dual recognition such as a PCR amplification or intramolecular circularization followed by rolling circle amplification, or to processes requiring single recognition such as array sorting and/or readout, balancing of sequence frequencies by hybridization to tag complements followed by elution, minisequencing, single molecule sequencing and various sequencing-by-synthesis strategies such 454 sequencing.

Since primed elongation, structure specific endonucleolytic cleavage and ligation of target sequence to the primary probe is dependent on sequence recognition this invention could also be used for assessment of small sequence variations such as Single Nucleotide Polymorphisms.

### Example

### PCR amplification of DNA fragment by use of common primer pair

### Step 1. Fragmentation

Fragmentation on human genomic DNA was carried out using restriction enzyme. 3 µl of DNA (1 µg/µl) was added to a solution of 0.4 U/µl CViA II and 1 X NEB buffer 4 in a total volume of 30 µl. The mixture was incubated at 25 °C for one h and then at 65 °C for 20 minutes to inactivate the restriction enzyme.

### Step 2. Ligation

5 µl of the mixture created in step 1 was added to a solution of 1 X Ampligase buffer, 0.2 U/µl Ampligase, 0.01 µg/µl Bovine Serum Albumine, 50 nM primary probe oligonucleotide, 100 pM tertiary probe and secondary probe oligonucleotide in a total volume of 15 µl. The mixture was incubated at 95 °C for 5 min, 75 °C for 10 min, 65 °C for 10 min, 60 °C for 10 min, 55 °C for 10 min and 50 °C for 10 min.

### Probe sequences

secondary probe and tertiary probe primary probe
AGCTTTCTACCGTTATCGT (SEQ ID NO: 2)

### Step 3. Extension

2.5 µl of the mixture from step 2 was added to a solution of 1X PCR buffer platinum, 200 µM dNTPs and 0.04 U/µl Taq polymerase in a total volume of 25 µl. The mixture was incubated at 55 °C for 5 min and then at 72 °C for 5 min.

### Step 4. PCR amplification

2.5 µl of the mixture from step 3 was added to 250 µM dNTPs, 0.9 X PCR buffer Platinum, 1.5 mM magnesium chloride, 100 nM forward primer, 100 nM reverse primer and 0.02 U/µl Platinum Taq polymerase in a final volume of 25 µl. The mixture was incubated at 95 °C for 5 min, thermo cycled 35 repeats between 95 °C for 30s, 55 °C for 30s and 72 °C for 1 min and thereafter incubated at 72 °C 2 min.

### Step 5. Agarose gel analysis

The samples, including one negative control, were loaded onto a 2% agarose gel and run for 50 minutes at 100V (Figure 7). The negative control was prepared as described in step 1-4 except that it is lacking the ligation substrate in the ligation mix. Lane 1 shows a 100 basepair DNA ladder, Lane 2 shows the negative control and lane 3 shows the product from the procedure described in step 1-4.

## Claims

1. A method for introducing common and/or individual sequence elements in a target nucleic acid molecule in a sample containing sample nucleic acid molecules, comprising the steps:
(i) if the sample nucleic acid molecules are double-stranded, denaturing the sample nucleic acid molecules, to obtain single-stranded sample nucleic acid molecules, wherein the 3'-ends of the sample nucleic acid molecules are not defined by one or more restriction endonucleases;
(ii) bringing the sample nucleic acid molecules in contact with primary, secondary and tertiary probe nucleic acid molecules,
wherein the 3'-end of the tertiary probe comprises a part complementary to the primary probe and the 5'-end of the tertiary probe comprises a part complementary to a 5'-part of the target nucleic acid molecule;
wherein the 3'-end of the secondary probe is complementary to a 3'-part of the target nucleic acid molecule, said 3'-part not including the 3'-end of the target molecule;
wherein the 5'-end of the secondary probe is not complementary to the target nucleic acid molecule; and
wherein said primary, secondary and tertiary probes comprise said common and/or individual sequence elements;
(iii) if the 5'-part of the target nucleic acid molecule that is complementary to the 5'-end of the tertiary probe does not include the 5'-end of the target nucleic acid molecule, cleaving off that part of the 5'-end of the target nucleic acid molecule that is not complementary to the tertiary probe;
(iv) ligating the 3'-end of the primary probe to the 5'-end of the target nucleic acid molecule; and
(v) elongating the 3'-end of the secondary probe by means of a nucleic acid polymerase.

2. The method according to claim 1, wherein the 5'-end of the secondary probe is linked to the 3'-end of the tertiary probe.

3. The method according to claim 1 or 2, further comprising prior to step (i) a step of fragmenting the sample nucleic acid molecules by either random fragmentation or sequence specific fragmentation.

4. The method according to any one of claims 1 to 3, wherein said common and/or individual sequence elements comprise a primer binding site, a tag sequence, a barcode sequence, a spacer, sites for enzymatic restriction digestion or sequence for sizecoding of fragments.

5. The method for selectively amplifying a target nucleic acid molecule in a sample comprising sample nucleic acid molecules, comprising the steps
- introducing sequence elements comprising amplification primer binding sites in the target nucleic acid molecule with the method according to claim 4; and
- amplifying the target nucleic acid molecule.

## Patentansprüche

1. Verfahren zum Einführen gemeinsamer und/oder individueller Sequenzelemente in ein Zielnucleinsäuremolekül in einer Probe, die Probennucleinsäuremoleküle enthält, umfassend die Schritte:
(i) wenn die Probennucleinsäuremoleküle doppelsträngig sind, Denaturieren der Probennucleinsäuremoleküle, um einzelsträngige Probennucleinsäuremoleküle zu erhalten, wobei die 3'-Enden der Probennucleinsäuremoleküle nicht durch eine oder mehrere Restriktionsendonucleasen definiert sind;
(ii) In-Kontakt-Bringen der Probennucleinsäuremoleküle mit primären, sekundären und tertiären Sondennucleinsäuremolekülen,
wobei das 3'-Ende der tertiären Sonde einen zu der primären Sonde komplementären Teil umfasst und das 5'-Ende der tertiären Sonde einen zu einem 5'-Teil des Zielnucleinsäuremoleküls komplementären Teil umfasst;
wobei das 3'-Ende der sekundären Sonde zu einem 3'-Teil des Zielnucleinsäuremoleküls komplementär ist, wobei der 3'-Teil das 3'-Ende des Zielmoleküls nicht einschließt;
wobei das 5'-Ende der sekundären Sonde zu dem Zielnucleinsäuremolekül nicht komplementär ist, und
wobei die primären, sekundären und tertiären Sonden die gemeinsamen und/oder individuellen Sequenzelemente umfassen;
(iii) wenn der 5'-Teil des Zielnucleinsäuremoleküls, der zum 5'-Ende der tertiären Sonde komplementär ist, das 5'-Ende des Zielnucleinsäuremoleküls nicht einschließt, Abspalten des Teils des 5'-Endes des Zielnucleinsäuremoleküls, der zu der tertiären Sonde nicht komplementär ist;
(iv) Ligieren des 3'-Endes der primären Sonde an das 5'-Ende des Zielnucleinsäuremoleküls, und
(v) Elongieren des 3'-Endes der sekundären Sonde mittels einer Nucleinsäure-Polymerase.

2. Verfahren gemäß Anspruch 1, wobei das 5'-Ende der sekundären Sonde mit dem 3'-Ende der tertiären Sonde verknüpft ist.

3. Verfahren gemäß Anspruch 1 oder 2, ferner umfassend vor Schritt (i) einen Schritt, bestehend im Fragmentieren der Probennucleinsäuremoleküle durch entweder zufällige Fragmentierung oder sequenzspezifische Fragmentierung.

4. Verfahren gemäß einem der Ansprüche 1 bis 3, wobei die gemeinsamen oder individuellen Sequenzelemente eine Primerbindungsstelle, eine Anhangssequenz, eine Barcode-Sequenz, einen Spacer, Stellen für enzymatischen Restriktionsverdau oder eine Sequenz zur Größenkodierung von Fragmenten umfassen.

5. Verfahren zum selektiven Amplifizieren eines Zielnucleinsäuremoleküls in einer Probe, die Probennucleinsäuremoleküle umfasst, umfassend die Schritte
- Einführen von Sequenzelementen, die Bindungsstellen für Amplifikationsprimer umfassen, in das Zielnucleinsäuremolekül mit dem Verfahren gemäß Anspruch 4, und
- Amplifizieren des Zielnucleinsäuremoleküls.

## Revendications

1. Procédé pour introduire des éléments de séquence communs et/ou individuels dans une molécule d'acide nucléique cible dans un échantillon contenant des molécules d'acide nucléique d'échantillon, comprenant les étapes suivantes :
(i) si les molécules d'acide nucléique d'échantillon sont bicaténaires, la dénaturation des molécules d'acide nucléique d'échantillon, pour obtenir des molécules d'acide nucléique d'échantillon monocaténaires, où les extrémités 3' des molécules d'acide nucléique d'échantillon ne sont pas définies par une ou plusieurs endonucléases de restriction ;
(ii) la mise en contact des molécules d'acide nucléique d'échantillon avec des molécules d'acide nucléique sondes primaires, secondaires et tertiaires,
où l'extrémité 3' de la sonde tertiaire comprend une partie complémentaire de la sonde primaire, et l'extrémité 5' de la sonde tertiaire comprend une partie complémentaire d'une partie en 5' de la molécule d'acide nucléique cible ;
où l'extrémité 3' de la sonde secondaire est complémentaire d'une partie en 3' de la molécule d'acide nucléique cible, ladite partie en 3' n'incluant pas l'extrémité 3' de la molécule cible ;
où l'extrémité 5' de la sonde secondaire n'est pas complémentaire de la molécule d'acide nucléique cible ; et
où lesdites sondes primaire, secondaire et tertiaire comprennent lesdits éléments de séquence communs et/ou individuels ;
(iii) si la partie en 5' de la molécule d'acide nucléique cible qui est complémentaire de l'extrémité 5' de la sonde tertiaire n'inclut pas l'extrémité 5' de la molécule d'acide nucléique cible, la séparation par clivage de cette partie de l'extrémité 5' de la molécule d'acide nucléique cible qui n'est pas complémentaire de la sonde tertiaire ;
(iv) la ligature de l'extrémité 3' de la sonde primaire avec l'extrémité 5' de la molécule d'acide nucléique cible ; et
(v) l'élongation de l'extrémité 3' de la sonde secondaire au moyen d'une polymérase d'acide nucléique.

2. Procédé selon la revendication 1, dans lequel l'extrémité 5' de la sonde secondaire est liée à l'extrémité 3' de la sonde tertiaire.

3. Procédé selon la revendication 1 ou 2, comprenant en outre avant l'étape (i) une étape de fragmentation des molécules d'acide nucléique d'échantillon soit par fragmentation aléatoire soit par fragmentation spécifique de séquence.

4. Procédé selon l'une quelconque des revendications 1 à 3, dans lequel lesdits éléments de séquence communs et/ou individuels comprennent un site de liaison d'amorce, une séquence étiquette, une séquence code-barres, un espaceur, des sites pour digestion par restriction enzymatique, ou une séquence pour codage de taille des fragments.

5. Procédé pour amplifier sélectivement une molécule d'acide nucléique cible dans un échantillon comprenant des molécules d'acide nucléique d'échantillon, comprenant les étapes suivantes
- introduction d'éléments de séquence comprenant des sites de liaison d'amorce d'amplification dans la molécule d'acide nucléique cible, par le procédé selon la revendication 4 ; et
- amplification de la molécule d'acide nucléique cible.


### SEQUENCE LISTING

<110> Johansson, Henrik Ericsson, olof
<120> Novel method
<130> P07864SE00/HAM
<150> SE0701401-2
   <151> 2007-06-11
<160> 2
<170> PatentIn version 3.3
<210> 1
   <211> 74
   <212> DNA
   <213> Artificial
<220>
   <223> Example: secondary and tertiary probe
<400> 1
   gagcccttat tgtactacat acgataacgg tagaaagctt tgctaacggt cgagggagag 60
   cagcttccag tata 74
<210> 2
   <211> 19
   <212> DNA
   <213> Artificial
<220>
   <223> Example: Primary probe
<400> 2
   agctttctac cgttatcgt 19